# EUROPEAN PATENT APPLICATION

(11) **EP 3 715 446 A1**
(43) Date of publication of application: **30.09.2020**
(21) Application number: 19382218.6
(22) Date of filing: 27.03.2019
(51) Int. Cl.: C12M 1/22, C12M 1/00, C12M 1/26

(54) **SCRAPERS FOR DETACHING CELLS, KITS AND METHODS**

(71) Applicant: Vallejo Blanco, Adrián, 31011 Pamplona (ES); Román Moreno, Marta, 23330 Villanueva del Arzobispo (ES); Sucunza Guibert, Diego, 31006 Pamplona (ES)
(72) Inventor: Vallejo Blanco, Adrián, 31011 Pamplona (ES); Román Moreno, Marta, 23330 Villanueva del Arzobispo (ES); Sucunza Guibert, Diego, 31006 Pamplona (ES)
(74) Representative: ZBM Patents - Zea, Barlocci & Markvardsen

(57) **Abstract**

The present disclosure relates to a scraper for detaching cells adhered to a bottom surface of a culture vessel is provided. The scraper comprises a scraping edge and a coupling surface for attaching the scraper to a bottom surface of a cover, wherein the cover is configured to be rotatably coupled to the culture vessel such that in use by rotating the cover with respect to the culture vessel, the scraper detaches the cells adhered to the bottom surface of the culture vessel. Kits including such scrapers and methods for detaching cells from a bottom surface of a culture vessel are also provided.

## Description

The present disclosure relates to scrapers for detaching cells adhered to a bottom surface of a culture vessel. The present disclosure further relates to kits including such scrapers and methods for detaching cells from a bottom surface of a culture vessel.

### BACKGROUND

Many laboratory procedures require the cultivation of cells for subsequent analysis and diagnostic tests. The cells are cultivated in cell culture vessels, such as flasks or petri dishes.

Particularly, a petri dish may include a bottom surface, a side wall enclosure and an open top. A cover may then be removably mounted to the open top of the side walls for selectively enclosing the interior of the cell culture vessel.

Culture vessels are employed by depositing a controlled amount of a liquid growth medium in the vessel. Cells that are to be cultivated are then deposited into the vessel. The vessel is closed by placing a cover over the open top defined by the side walls. Then, the vessel is stored in an environment that is conducive to cell growth. Cells growing in the vessel must be removed and analyzed periodically. The growing cells are likely to attach themselves to the bottom surface of the vessel, and hence must be scraped from the bottom surface for analysis.

Cell scrapers are used for removing cells from the bottom surface of a culture vessel. The typical cell scraper includes a handle and a scraper blade made from plastic material, wherein the blade is suitably coupled to the handle. The scraper blade generally includes a planar scraping edge

To properly scrape a culture, it may be necessary to access difficult to reach areas of the culture vessel by the cell scraper, such as corners of the vessel. Or in the case of a round culture vessel, the areas along the perimeter are hard to scrape using standard scrapers. The configuration of the scraper blade on the handle does not facilitate access to remote areas of the culture vessel. Furthermore, the blade and handle made of a rigid plastic are not ideal for removing cell cultures from parts of a surface that are difficult to reach.

Therefore, multiple passes must be performed with the scraper over the vessel in order to properly collect the adhered cells. Thus, the scraping of cells using the scraper can be time-consuming and it may be a cumbersome task to carry out. Also this scraper is a relative expensive solution. A further problem with a conventional cell scraper may be the risk of contamination during the removal of cells of the culture vessel. In summary, this known scraper is a highly inefficient design to properly collect cells adhered to the culturing vessel.

Examples of the present disclosure seek to at least partially reduce one or more of the aforementioned problems.

### SUMMARY

According to a first aspect, a scraper for detaching cells adhered to a bottom surface of a culture vessel is provided. The scraper comprises a scraping edge and a coupling surface for attaching the scraper to a bottom surface of a cover, wherein the cover is configured to be rotatably coupled to the culture vessel such that in use by rotating the cover with respect to the culture vessel, the scraper detaches the cells adhered to the bottom surface of the culture vessel

According to this first aspect, a scraper that is configured to provide the function of detaching cells from a bottom surface of a culture vessel is provided. To this end, the scraper comprises a coupling surface for attaching the scraper to a bottom surface of a cover. By the simple operation of rotating the cover (and thus the scraper) with respect to the culture vessel, cells can be easily detached from the bottom surface of the culture vessel.

This way, a scraper for detaching cells that is simple, cost-effective, and versatile and that can be used effectively with all types of cell culture vessels is provided.

In some examples, the scraper is made of silicone. The silicone may be able to flex and conform to surface irregularities of the cell culture vessel. However, the silicone of the scraping edge will return to or towards a straight shape for the scraping edge. Thus, the silicone of the scraping edge is well suited to scraping cell cultures from both easy and difficult to reach areas of the cell culture vessel. Additionally, the resilient characteristics of the silicone permit the maximum scraping edge to be in contact with the growth surface of the cells, and hence ensure more effective scraping of cell cultures.

In a further aspect, a kit is provided. The kit includes a scraper for detaching cells according to the first aspect. The kit further includes a substantially circular cover configured to be rotatably coupled to a substantially circular culture vessel.

In some examples, the kit may further be provided with a substantially circular culture vessel for cell cultivation. The vessel comprises a bottom surface, a circular sidewall and a culture space being formed in an interior of the culture vessel.

A kit comprising all or various of the elements needed in procedures for scraping cells located on a bottom surface of a culture vessel can thus be provided.

In yet another aspect, a method for detaching cells from a bottom surface of a culture vessel is provided. A scraper according to the first aspect is provided. A substantially circular culture vessel for cell cultivation comprising a bottom surface, a sidewall and a culture space being formed in an interior of the culture vessel is provided. Furthermore, a substantially circular cover configured to be rotatably coupled to the substantially circular culture vessel is provided. The cover and the culture vessel are brought in proximity to each other. The cover is aligned with the culture vessel. The cover is pushed towards the bottom surface of the culture vessel such that the cover is coupled to the culture vessel. Then, the cover is rotatably displaced with respect to the vessel such that cells located in the culture space and on the bottom surface of the vessel are scraped.

According to this aspect, the cover is coupled to the vessel. Moreover, the cover is rotatably displaced with respect to the vessel. The risk of contamination of the cells located in the culture vessel during the scraping process is thus reduced since the scraping process is performed with the cover coupled to the vessel e.g. in sterile conditions. Therefore, the use of e.g. a flow hood in order to achieve sterile conditions during the harvesting of cells may be avoided.

### BRIEF DESCRIPTION OF THE DRAWINGS

Non-limiting examples of the present disclosure will be described in the following, with reference to the appended drawings, in which:

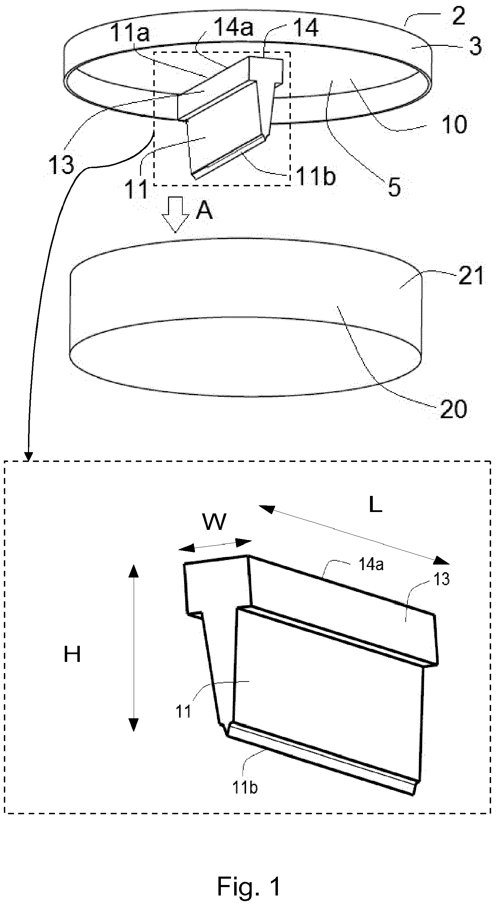

Figure 1 schematically illustrates an example of a culture vessel, a scraper for detaching cells from a bottom surface of the culture vessel and a cover.

### DETAILED DESCRIPTION OF EXAMPLES

Throughout the present description and claims, the term "bottom surface of a culture vessel" is used to refer to the growth surface of a cell culture vessel, i.e. the flat-surfaced floor wall of cell culture vessels, where cells grow and adhere and from which such cells need to be harvested, i.e. removed.

Details of the culturing method are not given, provided these are known from conventional methods for culturing biological cells for multiplication and / or differentiation purposes, particularly in culture vessel. Culturing conditions, particularly the selection of culturing media, the design of culturing protocols (the supply and removal of particular media according to a particular time plan) and physical conditions such as, for example, the temperature, pressure, air humidity and lighting can be provided, as known from conventional culturing methods.

Figure 1 schematically illustrates an example of a culture vessel, a scraper for detaching cells from a bottom surface of the culture vessel and a cover. Figure 1 schematically illustrates a three-dimensional view of a culture vessel, a scraper for detaching cells from a bottom surface of the culture vessel and a cover. In this example, a cover 2 is provided. The cover 2 in this example is substantially circular-shaped. The cover 2 comprises an outer circular sidewall 3 and a base 5. The base further comprises a bottom surface 10. The outer sidewall 3 may be of constant height as shown in this example. In some other examples now shown, the outer sidewall may comprise portions and the portions may be of varying height.

The cover 2 is configured to be rotatably coupled to a culture vessel 20, thus in use the cover closes off the top of the culture vessel 20. The cover 2 may have e.g. a threaded coupling with the culture vessel 20. In some examples, the sidewall 3 of the cover 2 may flare outwardly slightly so as to facilitate coupling the cover 2 on to the culture vessel 20. Moreover, the cover 2 may be sufficiently large in diameter in order to be rotatably coupled to the vessel 20 in a suitable way. The cover 2 may be formed of any suitable material e. g. metal, synthetic resin, glass, a polymeric material or the like, being preferably a non-porous and relatively non-fragile material capable of withstanding sterilization.

The cover 2 may comprise a grip (not shown) located e.g. at the outer part of the sidewall of the cover, thus the attachment, removal and manipulation of the cover 2 (and thus a scraper 11 attached to the cover) may be improved. The grip may be shaped so that it may be easily held between the fingers of a user. The grip may also be formed by a certain surface roughness, optionally provided by a coating. With this arrangement, the cover 2 may be efficiently manipulated and used with great accuracy. Particularly, the cover 2 may be easily attached and rotated with respect to the culture vessel 20 such that cells located on a bottom surface of the culture vessel 20 are readily scraped away.

Furthermore, a scraper 11 is provided (see enlarged detail of figure 1). The scraper may extend from a proximal end 11a (closer to the person handling the device 1) to the distal end 11b (the end further away from the person handling the device 1). The scraper 11 may comprise a coupling surface 14 for mounting the scraper 11 to the inner surface 10 of the cover 2. The scraper 11 may be removably mounted to the inner surface 10. In examples, the scraper 11 may be integrally formed with the cover 2.

Additionally, a mounting element 13 integrally formed with the scraper 11 may be provided. The mounting element 13 may be wider than the scraper blade and thereby provides more surface area for the coupling surface 14a. In consequence, an improved attachment of the scraper 11 to the inner surface 10 of the cover 2 is achieved. In some alternative examples, a separate mounting element 13 may be provided to which the scraper blade can be (removably) attached.

In some examples, the coupling surface 14 of the scraper 11 may further be provided with an adhesive for sticking the scraper 11 to the inner surface of the cover 2. The procedure to attach and / or remove the scraper from the inner surface of cover 2 may thus be improved.

In this particular example, the scraper 11 defines a substantially vertical plane of symmetry, and once attached, the vertical plane of the scraper may be arranged with respect to the inner surface 10 of the cover 2 at an angle of approximately 90 degrees. However, the scraper may be tilted at different angles with respect to the plane defined by the inner surface 2 of the cover in order to achieve different scraping effects.In other examples, the scraper may define itself a substantially vertical plane.

Additionally, the scraper 11 is provided with a scraping edge 11b. In some examples, the scraping edge 11b may be a flexible scraping edge 11b configured to be deformed such that the scraping edge 11b conforms to the specific contour of the inner surface of the culture vessel 20.

In this particular example, the scraper 11 may be V-shaped in cross-section. The scraper 11 may be constructed of a flexible and resilient material allowing the scraping edge 11b to be bendable from its generally straight configuration. The scraping edge 11b resiliently returns to its straight configuration in the absence of any bending forces. The flexible and resilient material may be silicone or silicone rubber, although some other soft-plastic materials may be possible.

The scraper may have a width W between 5 and 10 mm. The scraper may further have a length L between 50 and 75 mm, and a height H between 16 and 26 mm.

Moreover, the scraping edge may also have different textures and different edges profiles e.g. jagged, sawtooth. In some non-illustrated examples, multiple scraper edges may be provided on a single scraper to further increase the efficiency of the scraper 11.

In this particular example, the scraper 11 and the scraping edge 11b may be integrally formed. By constructing the scraper 11 and the scraping edge 11b as a simple one piece, assembly is not required, thus mass and rapid fabrication of the scraper by methods such as injection, extrusion, moulding, 3D printing, stamping, and casting is facilitated.

Wear and tear of the scraper 11 through use may cause the scraping edge 11b to dull, and also cause cavities and recesses to form over the scraping edge 11b. A dull scraping edge may reduce the effectiveness of the scraping action, and cavities and recesses on the scraping edge encourage the trapping of unwanted debris. Thus, in examples, the scraping edge 11b may be constructed as a disposable item. Additionally, the scraper 11 (and the remaining elements forming the scraper) may be treated using sterilizing procedures to keep the scraper free of biological organisms

The culture vessel 20 shown in this example comprises a bottom inner surface (not visible) and a sidewall 21. The sidewall 21 and the inner surface enclose a culturing space.

The scraper 11 may be sterilized and packaged in a sterile packaging, e.g. a wrap. Enclosed with the scraper 11, a culture vessel 20 and / or a cover as hereinbefore described may further be provided.

In accordance with an aspect, the cells can be detached from a bottom surface of the culture vessel substantially as follows:
A culture vessel may be provided. The culture vessel 20 may be placed in e.g. an incubator (not shown) whose internal temperature and humidity are kept constant. The culture vessel 20 contains a culture medium, which is a liquid for culturing cells. Cells (adherent cells) adhere to the inner bottom surface (not visible) of the culture vessel 20. The culturing vessel 20 may be removed from the incubator with cells cultivated on the bottom inner surface of the culture vessel.

Furthermore, a scraper packaged in a sterile packaging as hereinbefore described may be provided. The scraper is adapted to detach cells adhered to the bottom surface of the culture vessel 20.

Additionally, a cover 2 may be provided. When the culture vessel is removed from the incubator, the sterile packaging is opened. In this case, a user may attach the scraper 11 to the inner surface of the cover 2 in preparation for use. In some other examples, the scraper 11 may be pre-assembled with the cover 2, thus forming a pre-assembled device.

The cover 2 (and thus the scraper 11 attached to the cover) may be brought into proximity of the culture vessel 20. The cover 2 may further be aligned with the culture vessel 20 and attached to the culture vessel 20, thus closing off the culture vessel 20. Once the cover 2 is attached to the culture vessel 20, the scraper 11 (and thus the scraping edge 11b) may be situated over the bottom surface (not visible) of the culture vessel 20 in preparation for use. A user may apply a force in the direction of the arrow (arrow A) using the grip portion of the cover 2, typically by forcing the cover 2 towards the vessel 20. In some examples, the force may slightly bend the scraping edge. This way, the scraping edge 11b is deformed such that the edge conforms to the specific contour of the bottom surface of the culture vessel 20

The cover 2 (and thus the scraper 11 and the blade 11b) may be rotated with respect to the culture vessel 20 such that the cells previously cultivated and adhered to the inner surface of the culture vessel 20 are removed. The scraper is moved along the inner bottom surface of the culture vessel while being pushed against the inner surface of vessel by the user. The length of the scraper may be set to be at least equal to the radius of the substantially circular inner bottom surface of the vessel 20. This process is repeated as desired.

Once the cells are detached from the inner surface of the vessel 20, the cover can be removed and the cells can be collected from the vessel surface 20. The collection of the cells from the vessel surface 20 may be performed using e.g. a pipette device (not shown). The pipette device may suck cells detached from the inner surface of the vessel together with the culture medium.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

## Claims

1. A scraper for detaching cells adhered to a bottom surface of a culture vessel, wherein the scraper comprises:
• a scraping edge, and
• a coupling surface for attaching the scraper to a bottom surface of a cover, wherein the cover is configured to be rotatably coupled to the culture vessel such that in use by rotating the cover with respect to the culture vessel, the scraper detaches the cells adhered to the bottom surface of the culture vessel.

2. A scraper according to claim 1, further comprising a mounting element for carrying the scraper and the mounting element including the coupling surface, and optionally wherein the scraper is integrally formed with the mounting element.

3. A scraper according to any of claims 1 - 2, wherein the scraper comprises a substantially straight scraping edge.

4. A scraper according to any of claims 1 - 3, wherein the scraper is made of silicone.

5. A scraper according to any of claims 1 - 4, wherein the scraper defines a substantially vertical plane, and in use, the vertical plane of the scraper is arranged at an angle of 90 degrees with respect to the plane defined by the inner surface of the cover.

6. A scraper according to any of claims 1 - 5, wherein the coupling surface of the scraper comprises an adhesive for sticking the scraper to the inner surface of the cover.

7. A scraper according to any of claims 1 - 6, in a sterile package.

8. A kit including:
• a scraper according to any of claims 1 - 7,
• a substantially circular cover configured to be rotatably coupled to a substantially circular culture vessel.

9. A kit according to claim 8, further comprising a substantially circular culture vessel for cell cultivation comprising a bottom surface, a circular sidewall, and a culture space being formed in an interior of the culture vessel.

10. A kit according to claim 9, wherein the cover comprises first coupling elements, and the culture vessel comprises second coupling elements that are adapted to mate with the first coupling elements, and wherein the coupling elements are configured for an axial movement of the cover with respect to the vessel during coupling.

11. A kit according to any of claims 9 - 10, wherein the cover further comprises a grip configured to manipulate the cover.

12. A kit according to any of claims 8 - 11, wherein the scraper is integrally formed with the cover.

13. A method for detaching cells from a bottom surface of a culture vessel comprising:
- providing a scraper according to any of claims 1 - 7;
- providing a substantially circular culture vessel for cell cultivation comprising a bottom surface, a sidewall and a culture space being formed in an interior of the culture vessel;
- providing a substantially circular cover configured to be rotatably coupled to the substantially circular culture vessel;
- bringing the cover and the culture vessel in proximity of each other;
- aligning the cover with the culture vessel;
- pushing the cover towards the bottom surface of the culture vessel such that cover is coupled to the culture vessel;
- rotatably displacing the cover with respect to the vessel such that cells located in the culture space and on the bottom surface of the vessel are scraped using the scraper.

14. A method for detaching according to claim 13, before bringing the cover and the culture vessel in proximity of each other, attaching the coupling surface of the scraper to the inner surface of the cover.

15. A method according to any of claims 13 - 14, further comprising before bringing the cover and the culture vessel in proximity of each other:
- providing a culture medium into the culture space of the culture vessel,
- inoculating the culture medium with cells, and
- propagating the cells in the culture medium.
